# EUROPEAN PATENT APPLICATION

(11) **EP 4 563 102 A2**
(43) Date of publication of application: **04.06.2025**
(21) Application number: 25165220.2
(22) Date of filing: 04.02.2009
(51) Int. Cl.: A61B 17/34

(54) **CROSSING OCCLUSIONS IN BLOOD VESSELS**

(30) Priority: 05.02.2008 US 6375608 P
(62) Divisional of application: 20199215.3
(71) Applicant: Boston Scientific Scimed, Inc., Maple Grove, MN 55311 (US)
(72) Inventor: The designation of the inventor has not yet been filed
(74) Representative: Vossius & Partner Patentanwälte Rechtsanwälte mbB

(57) **Abstract**

An apparatus for facilitating treatment via a vascular wall (22) defining a vascular lumen (30) containing an occlusion (36) therein, the apparatus comprising: an intravascular device (106) having a lumen (122) and a distal portion, the distal portion including a first aperture (126) and a second aperture (128) that are both in fluid communication with the lumen (122); and a reentry device (100) having a body (138) and a penetrator (140) extending distally beyond a distal end (132) of the body (138), wherein the body (138) is biased to assume a bent shape including a bend (130) in a natural state, wherein the body (138) has a distal leg (144) disposed distally of the bend (130) and a proximal leg (146) disposed proximally of the bend (130); wherein the penetrator (140) extends beyond the distal end (132) of the body (138) by a distance (L1), and the distal leg (144) has a length (L2), wherein the length (L2) is greater than the distance (L1).

## Description

### Cross Reference to Related Application

This application claims the benefit of U.S. Provisional Application No. 61/063,756, filed February 5, 2008, which is herein incorporated by reference in its entirety.

### Field of the Invention

The inventions described herein relate to devices and associated methods for the treatment of chronic total occlusions. More particularly, the inventions described herein relate to devices and methods for crossing chronic total occlusions and establishing a pathway for blood flow past the chronic total occlusions.

### Background of the Invention

Due to age, high cholesterol and other contributing factors, a large percentage of the population has arterial atherosclerosis that totally occludes portions of the patient's vasculature and presents significant risks to patient health. For example, in the case of a total occlusion of a coronary artery, the result may be painful angina, loss of cardiac tissue or patient death. In another example, complete occlusion of the femoral and/or popliteal arteries in the leg may result in limb threatening ischemia and limb amputation.

Commonly known endovascular devices and techniques are either inefficient (time consuming procedure), have a high risk of perforating a vessel (poor safety) or fail to cross the occlusion (poor efficacy). Physicians currently have difficulty visualizing the native vessel lumen, can not accurately direct endovascular devices toward the visualized lumen, or fail to advance devices through the lesion. Bypass surgery is often the preferred treatment for patients with chronic total occlusions, but less invasive techniques would be preferred.

Described herein are devices and methods employed to exploit the vascular wall of a vascular lumen for the purpose of bypassing a total occlusion of an artery. Exploitation of a vascular wall may involve the passage of an endovascular device into and out of said wall which is commonly and interchangeable described as false lumen access, intramural access, submedial access or in the case of this disclosure, subintimal access.

### SUMMARY OF THE INVENTION

Described herein are devices and methods employed to exploit the vascular wall of a vascular lumen for the purpose of bypassing a total occlusion of an artery. Exploitation of a vascular wall may involve the passage of an endovascular device into and out of said wall which is commonly and interchangeable described as false lumen access, intramural access, submedial access or in the case of this disclosure, subintimal access.

In one aspect, the present disclosure is directed a method of facilitating treatment via a vascular wall defining a vascular lumen containing an occlusion therein. The method may include providing a first intravascular device having a distal portion and at least one aperture and positioning the distal portion of the first intravascular device in the vascular wall. The method may further include providing a reentry device having a body and a distal tip, the distal tip having a natural state and a compressed state and inserting the distal tip, in the compressed state, in the distal portion of the first intravascular device. The method may further include advancing the distal tip, in the natural state, through the at least one aperture of the first intravascular device.

In another aspect, the present disclosure is directed an apparatus for facilitating treatment via a vascular wall defining a vascular lumen containing an occlusion therein. The apparatus may include a first intravascular device having a distal portion, the distal portion including at least one aperture, at least one radiopaque marker, and at least one orienting element. The apparatus may further include a reentry device having a body and a distal tip, the distal tip having a natural state and a compressed state.

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

The accompanying drawings, which are incorporated in and constitute a part of this specification, illustrate several embodiments of the invention and together with the description, serve to explain the principles of the invention.

### BRIEF DESCRIPTION OF THE DRAWINGS

Figure 1 is a cross-sectional view of an artery with a wall having three layers. The outermost layer of the wall is the adventitia and the innermost layer is the intima. The tissues extending between the intima and the adventitia may be collectively referred to as the media.
Figure 2 is an additional view of the artery shown in the previous figure in which a crossing device has been advanced over guidewire so that a distal portion of the crossing device is disposed in a proximal segment of a true lumen of the artery.
Figure 3 is an additional view of the artery shown in the previous figure in which the distal end of the crossing device has been advanced in a distal direction so that a tip of the crossing device is adjacent to an occlusion.
Figure 4 is an additional view of the artery and crossing device shown in the previous figure. In the embodiment of figure 4, the distal end of the crossing device has been advanced in an axial direction past the occlusion.
Figure 5 is a view of the artery shown in the previous figure showing that the crossing device has been withdrawn from the true lumen of the artery.
Figure 6 is an additional view of the artery and the guidewire shown in the previous figure. In the embodiment of figure 6, an orienting device has been advanced over the guidewire.
Figure 7 is an additional view of the artery and the orienting device shown in the previous figure. In the embodiment of figure 7, the guidewire has been withdrawn leaving the orienting device in the position shown in figure 7.
Figure 8 is an additional view of the artery and the orienting device shown in the previous figure. In the embodiment of figure 8, a re-entry device has been advanced into the lumen of the orienting device.
Figure 9 is an enlarged partial cross-sectional view showing a portion of the re-entry device and the orienting device shown in the previous figure.
Figure 10 is an additional partial cross-sectional view showing a portion of the re-entry device and the orienting device shown in the previous figure.
Figure 11 is an enlarged partial cross-sectional view showing a portion of the re-entry device and the orienting device shown in the previous figure.
Figure 12 is another enlarged partial cross-sectional view showing a portion of the re-entry device shown in the previous figure. In the embodiment of figure 12, a core of the re-entry device has been advanced so that a portion of the core extends beyond the body of the re-entry device.
Figure 13 is an additional enlarged partial cross-sectional view showing a portion of the re-entry device shown in the previous figure. In the embodiment of figure 13, the re-entry device has been advanced so that a penetrator is contacting the intima of an artery.
Figure 14 is yet another enlarged partial cross-sectional view showing a portion of the re-entry device shown in the previous figure. In the embodiment of figure 14, the penetrator of the re-entry device has pierced the intima.
Figure 15 is still another enlarged partial cross-sectional view showing a portion of the re-entry device shown in the previous figure. In the embodiment of figure 15, the re-entry device has been advanced so that the distal end of the re-entry device is disposed in the true lumen of an artery.
Figure 16 is a partial cross-sectional view of the re-entry device shown in the previous figure. Figure 16 has a different scale than the previous figure so that more of the surrounding context is visible in figure 16.
Figure 17 is an additional view of the artery shown in the previous figure. In the embodiment of figure 17, an orienting device has been withdrawn leaving a re-entry device in the position shown in figure 17.
Figure 18 is a plan view of a crossing device in accordance with the present description. In the embodiment of figure 19, a penetrator of the crossing device is assuming a deployed position.
Figure 19 is an additional plan view of the re-entry device shown in the previous figure. In the embodiment of figure 19, a penetrator of the crossing device is assuming a retracted position.
Figure 20 is a plan view of an exemplary re-entry device. Selected dimensions of the exemplary re-entry device are illustrated in figure 20.
Figure 21 and figure 22 illustrate a method in which a re-entry device has been advanced while a core is in a retracted position. These figures show that the re-entry device has not penetrated intima. Instead, the re-entry device has been advanced between the intima and the exterior of the orienting device.
Figure 23A is a cross-sectional view of an orienting device including a radiopaque marker.
Figure 23B is a representation of a fluoroscopic display. The radiopaque marker of the orienting device shown in the previous figure is visible in this fluoroscopic display. A radiopaque re-entry device is also visible in this display.
Figure 24 is a plan view including the orienting device shown in the previous figure. In figure 24, a distal portion of a re-entry device can be seen extending through an aperture of the orienting device.
Figure 25A is a cross-sectional view of an orienting device including a radiopaque marker.
Figure 25B is a representation of a fluoroscopic display. The radiopaque marker of the orienting device shown in the previous figure is visible in this fluoroscopic display. A radiopaque re-entry device is also visible in this display.
Figure 26 is a plan view including the orienting device shown in the previous figure. A first balloon and a second balloon of the orienting device are visible in figure 26.
Figure 27 is a cross sectional view of the orienting device shown in the previous figure. A cut of the cross-sectional view is taken along line A-A shown in Figure 27.
Figure 28 is a cross-sectional view of an artery with a wall having three layers. The outermost layer of the wall is the adventitia and the innermost layer is the intima. In figure 28, an orienting device is shown disposed between the adventitia and the intima.
Figure 29 is a partial cross-sectional view of an exemplary crossing device comprising a tip that is fixed to a distal end of a shaft.
Figure 30 is a plan view showing an assembly including the crossing device shown in the previous figure. In the embodiment of Figure 30, a drive assembly is coupled to the shaft of the crossing device.
Figure 31 is a cross-sectional view of the assembly shown in the previous figure. The assembly includes a drive assembly comprising a central gear, an internal gear and a plurality of planetary gears. These gears form a gear train that provides a mechanical advantage.

### DESCRIPTION OF THE EMBODIMENTS

Reference will now be made in detail to the present embodiments of the invention, examples of which are illustrated in the accompanying drawings. Wherever possible, the same reference numbers will be used throughout the drawings to refer to the same or like parts.

The following detailed description should be read with reference to the drawings in which similar elements in different drawings are numbered the same. The drawings, which are not necessarily to scale, depict illustrative embodiments and are not intended to limit the scope of the invention.

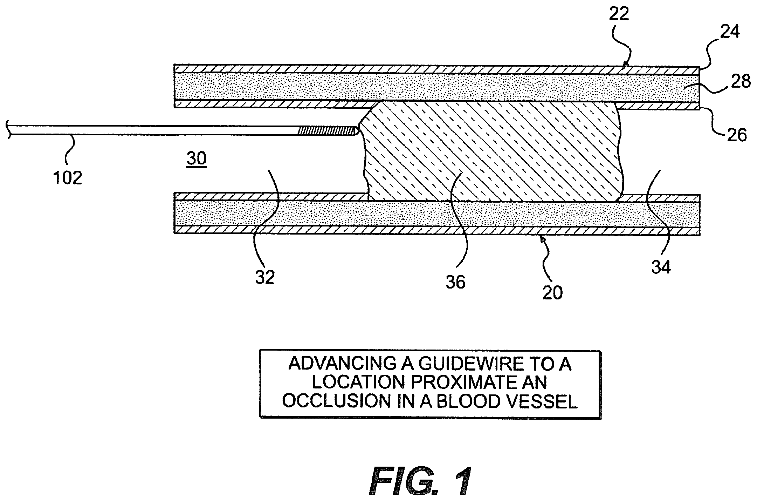

Figure 1 is a cross-sectional view of an artery 20 having a wall 22. In figure 1, wall 22 of artery 20 is shown having three layers. The outermost layer of wall 22 is the adventitia 24 and the innermost layer of wall 22 is the intima 26. The tissues extending between intima 26 and adventitia 24 may be collectively referred to as the media 28. For purposes of illustration, intima 26, media 28 and adventitia 24 are each shown as a single homogenous layer in figure 1. In the human body, however, the intima and the media each comprise a number of sublayers. The transition between the external most portion of the intima and the internal most portion of the media is sometimes referred to as the subintimal space. Intima 26 defines a true lumen 30 of artery 20. In figure 1, an occlusion 36 is shown blocking true lumen 30. Occlusion 36 divides true lumen 30 into a proximal segment 32 and a distal segment 34. In figure 1, a distal portion of a guidewire 102 is shown extending into proximal segment 32 of true lumen 30.

As shown in figure 1, methods described in this document may include the step of advancing a guidewire to a location proximate an occlusion in a blood vessel. The exemplary methods described in this document may also include the step of advancing guidewire 102 between occlusion 36 and adventitia 24. In some cases, however, the nature of the occlusion and the blood vessel will be such that the guidewire is unlikely to advance beyond the occlusion.

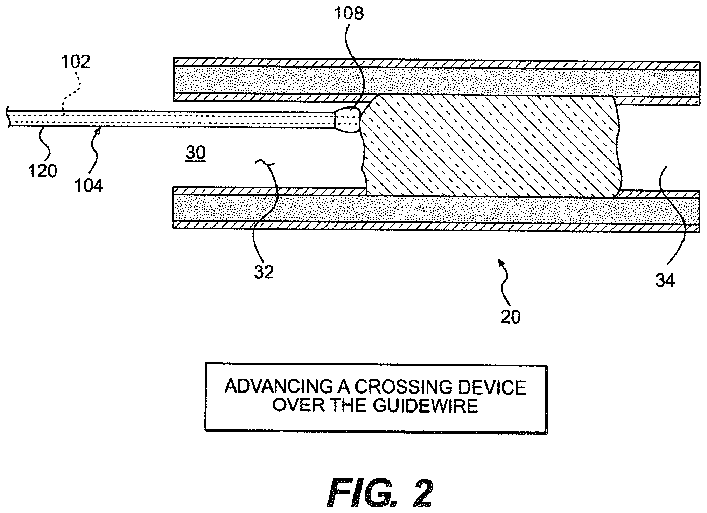

Figure 2 is an additional view of artery 20 shown in the previous figure. In the embodiment of figure 2, a crossing device 104 has been advanced over guidewire 102 so that a distal portion of crossing device 104 is disposed in proximal segment 32 of true lumen 30. Crossing device 104 may be used to establish a channel between proximal segment 32 and distal segment 34. Crossing device 104 of figure 2 comprises a tip 108 that is fixed to a distal end of a shaft 120. As shown in figure 2, methods described in this document may include the step of advancing a crossing device over a guidewire.

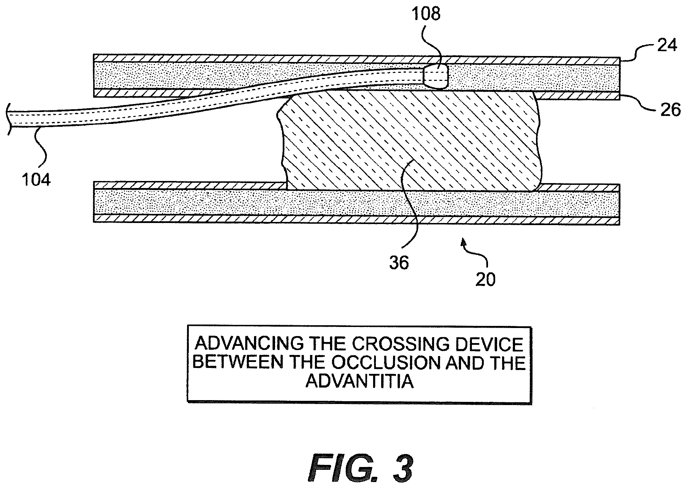

Figure 3 is an additional view of artery 20 shown in the previous figure. In the embodiment of figure 3, the distal end of crossing device 104 has been advanced in a distal direction so that tip 108 is adjacent to occlusion 36. With reference to figure 3, it will be appreciated that tip 108 has passed through intima 26 and is disposed between occlusion 36 and adventitia 24 of artery 20. Some methods described in this document may include the step of advancing a crossing device between an occlusion and the adventitia of an artery.

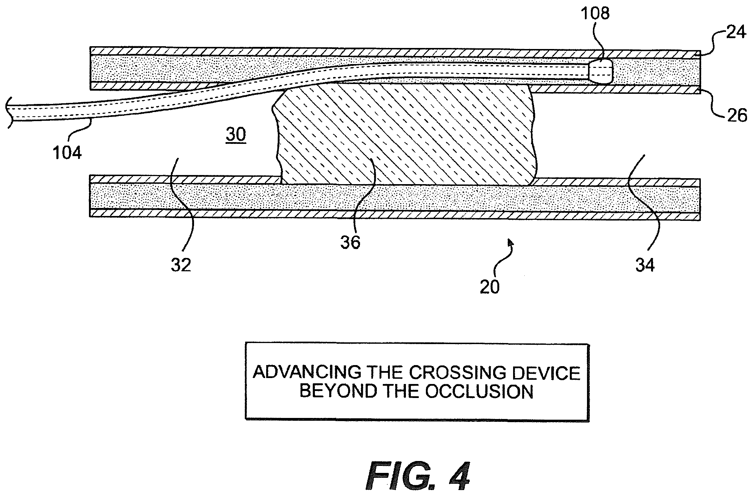

Figure 4 is an additional view of artery 20 and crossing device 104 shown in the previous figure. In the embodiment of figure 4, the distal end of crossing device 104 has been advanced in an axial direction past occlusion 36. Accordingly, it will be appreciated that methods described in this document may include the step of advancing a crossing device beyond an occlusion.

In the embodiment of figure 4, crossing device has crossed occlusion 36 by advancing between occlusion 36 and adventitia 24 of artery 20. It is to be appreciated that other methods of crossing an occlusion are within the spirit and scope of this disclosure. For example, the crossing device 104 may pass through occlusion 36 while remaining disposed inside true lumen 30.

In figure 4, tip 108 of crossing device 104 is shown residing between intima 26 and adventitia 24 of artery 20. As tip 108 moves in an axial direction between intima 26 and adventitia 24, tip 108 may cause blunt dissection of the layers forming the wall of artery 20. Alternatively, tip 108 may cause blunt dissection of the materials comprising the occlusion 36.

In some useful methods in accordance with the present disclosure, crossing device 104 is rotated about its longitudinal axis and moved in a direction parallel to its longitudinal axis simultaneously. When this is the case, rotation of crossing device 104 may reduce resistance to the axial advancement of crossing device 104. These methods take advantage of the fact that the kinetic coefficient of friction is usually less than the static coefficient of friction for a given frictional interface. Rotating crossing device 104 assures that the coefficient of friction at the interface between the crossing device and the surround tissue will be a kinetic coefficient of friction and not a static coefficient of friction.

With reference to figure 4, it will be appreciated that crossing device 104 extends past occlusion 36. In figure 4, occlusion 36 is shown blocking a true lumen 30. Occlusion 36 divides true lumen 30 into a proximal segment 32 and a distal segment 34. When a crossing device in accordance with some embodiments of the present disclosure is advanced through the subintimal space of an artery, the distal end of the crossing device may penetrate the intima and enter the distal segment of the true lumen after advancing beyond an occlusion. When this is the case, fluid communication between the proximal segment and the distal segment may be achieved via a channel created by the crossing device.

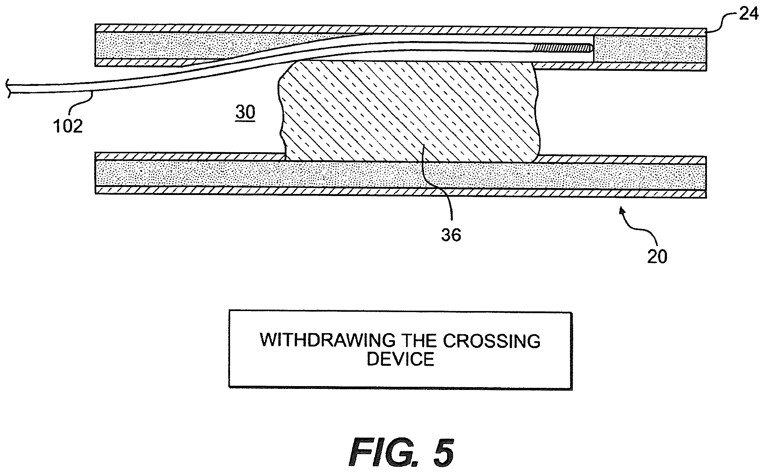

Figure 5 is an additional view of artery 20 shown in the previous figure. In the embodiment of figure 5, crossing device 104 has been withdrawn from true lumen 30 of artery 20. With reference to figure 5, it will be appreciated that guidewire 102 remains in the position formerly occupied by crossing device 104.

The position of guidewire 102 shown in figure 5 may be achieved using crossing device 104. Guidewire 102 may be positioned, for example, by first placing crossing device 104 in the position shown in the previous figure, then advancing guidewire 102 through lumen 122 defined by shaft 120 of crossing device 104. Alternately, guidewire 102 may be disposed within lumen 122 while crossing device 104 is advanced beyond occlusion 36.

With guidewire 102 in the position shown in figure 5, guidewire 102 may be used to direct other devices between occlusion 36 and adventitia 24. For example, a catheter may be advanced over guidewire 102 until the distal end of the catheter extends between an occlusion and the adventia. After reaching this location, the catheter may be used to dilate the tissue surrounding the catheter. Examples of catheters that may be used to dilate tissue include balloon catheters and atherectomy catheters.

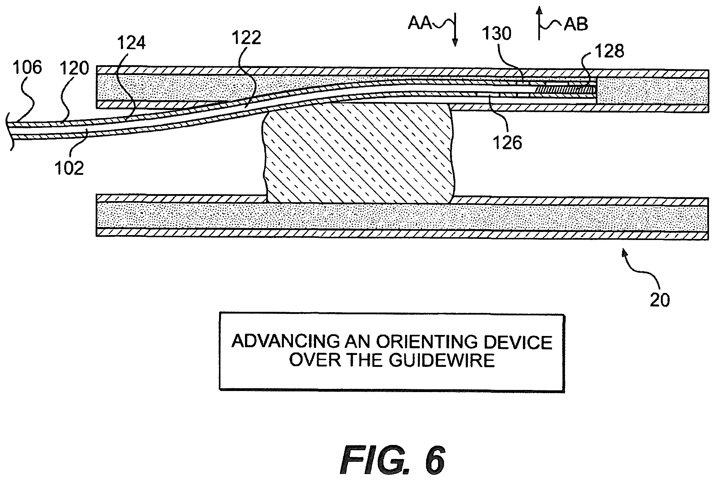

Figure 6 is an additional view of artery 20 and guidewire 102 shown in the previous figure. In the embodiment of figure 6, an orienting device 106 has been advanced over guidewire 102. Orienting device 106 includes a shaft 120 comprising a wall 124 defining a lumen 122. A first aperture 126 and a second aperture 128 are also defined by wall 124 . In the embodiment of figure 6, first aperture 126 and second aperture 128 are both in fluid communication with lumen 122.

In the embodiment of figure 6, orienting device 106 has been positioned so that first aperture 126 opens toward intima 26 of artery 20 and second aperture 128 opens toward adventitia 24. With reference to figure 6, it will be appreciated that first aperture extends in a first direction that is represented by a first arrow AA and second aperture extends in a second direction that is represented by a second arrow AB.

In figure 6, first arrow AA and second arrow AB are used to illustrate the fact that the second direction is general opposite the first direction. In the embodiment of figure 6, first arrow AA and second arrow AB are orient 180 degrees away from each other. In the embodiment of figure 6, first aperture 126 and second aperture 128 are longitudinally separated from one another. Orienting device 106 includes a radiopaque marker 130 that is located between first aperture 126 and second aperture 128.

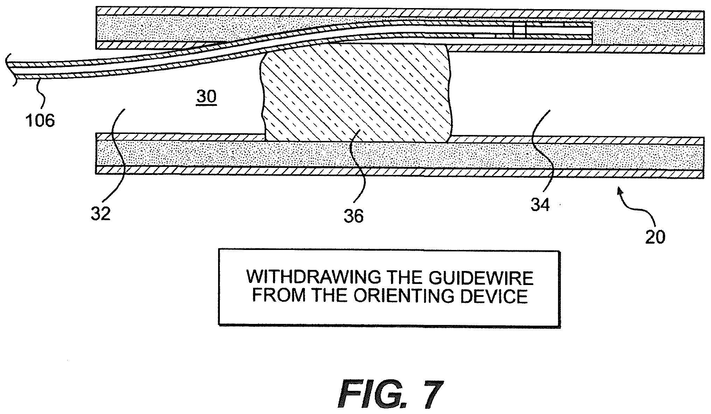

Figure 7 is an additional view of artery 20 and orienting device 106 shown in the previous figure. In the embodiment of figure 7, guidewire 102 has been withdrawn leaving orienting device 106 in the position shown in figure 7. With reference to figure 7, it will be appreciated that orienting device 106 extends beyond occlusion 36. In figure 7, occlusion 36 is shown blocking true lumen 30. Occlusion 36 divides true lumen 30 into a proximal segment 32 and a distal segment 34. When an orienting device in accordance with some embodiments disclosed herein is advanced between the adventitia and the intima of an artery, the orienting device may be used to direct a re-entry device toward true lumen 30. Fluid communication between proximal segment 32 and distal segment 34 may be achieved by re-entering the true lumen with the re-entry device.

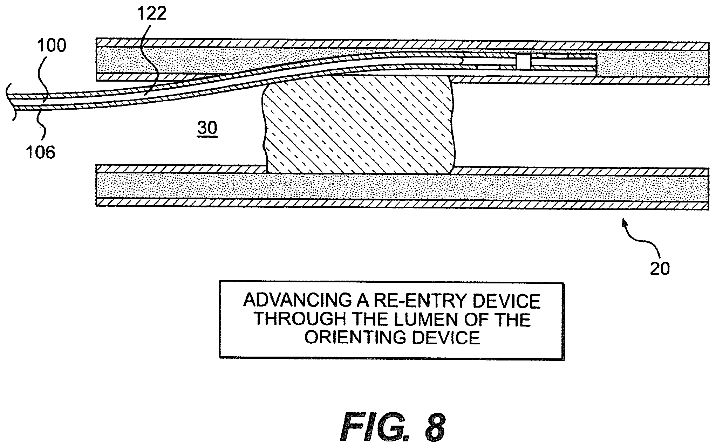

Figure 8 is an additional view of artery 20 and orienting device 106 shown in the previous figure. In the embodiment of figure 8, a re-entry device 100 has been advanced into lumen 122 of orienting device 106. Some useful methods include the step of advancing the distal end of re-entry device 100 into true lumen 30.

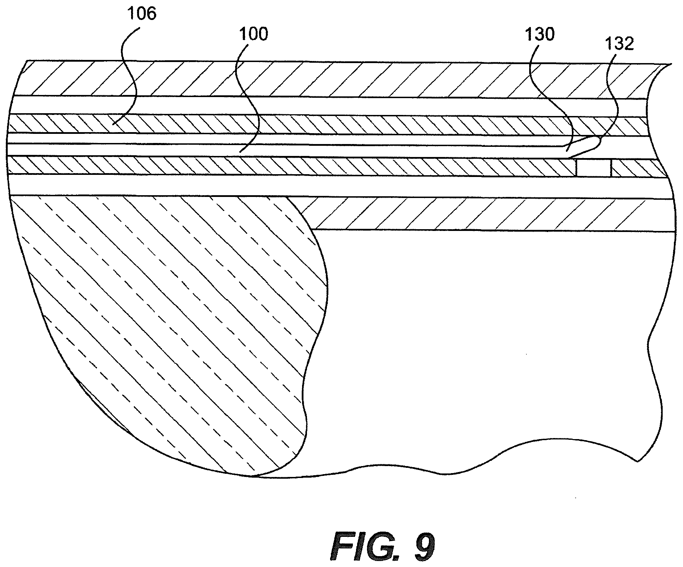

Figure 9 is an enlarged partial cross-sectional view showing a portion of re-entry device 100 and orienting device 106 shown in the previous figure. With reference to figure 9, it will be appreciated that re-entry device 100 includes a bend 130 near distal end 132 of re-entry device 100.

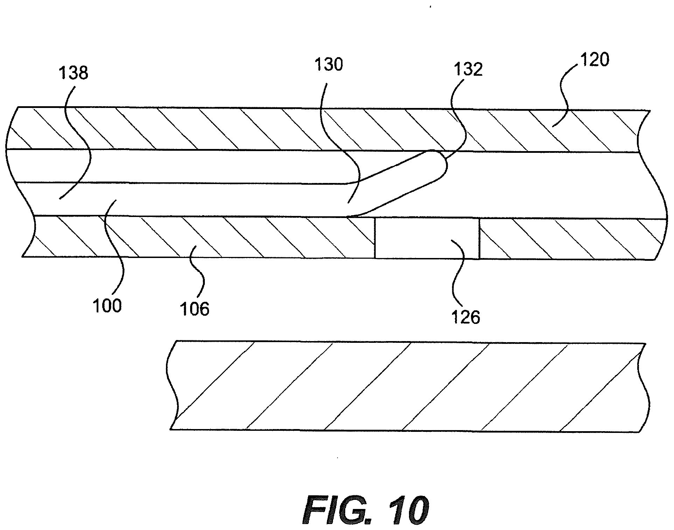

Figure 10 is an additional partial cross-sectional view showing a portion of re-entry device 100 and orienting device 106. Figure 10 is further enlarged and simplified relative to the items shown in the previous figure. In the embodiment of figure 10, a body 138 of re-entry device 100 is biased to assume a bent shape including a bend 130. Also in the embodiment of figure 10, shaft 120 of orienting device 106 is holding re-entry device 100 in a somewhat compressed state. When this is the case, re-entry device 100 can be inserted through first aperture 126 by positioning distal end 132 over first aperture 126 and allowing bend 130 to assume it's natural state (i.e., bent at a sharper angle). Re-entry device 100 can be inserted through aperture 126 until it comes into contact with intima 26.

It the embodiment of figure 10, distal end 132 of core 136 is axially aligned with first aperture 126, however, bend 130 is causing distal end 132 to point away from first aperture 126. When this is the case, distal end 132 may be positioned over first aperture 126 by rotating core 136.

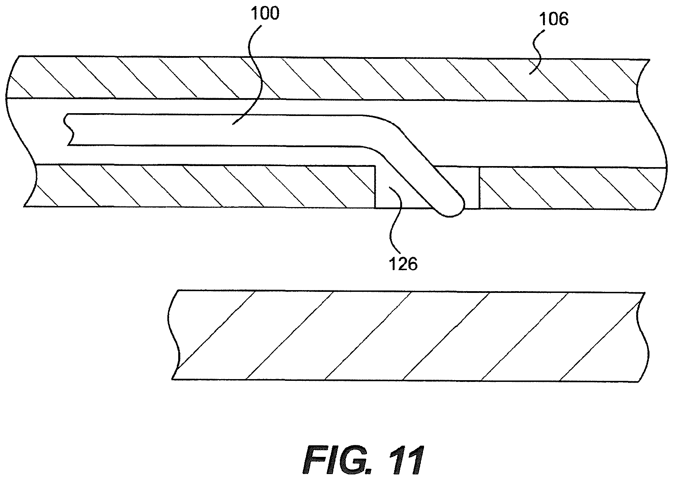

Figure 11 is an enlarged partial cross-sectional view showing a portion of re-entry device 100 and orienting device 106 shown in the previous figure. In the embodiment of figure 11, re-entry device 100 has been positioned so that a distal portion of re-entry device 100 has entered first aperture 126. Intima 26 is shown below first aperture 126 in figure 11.

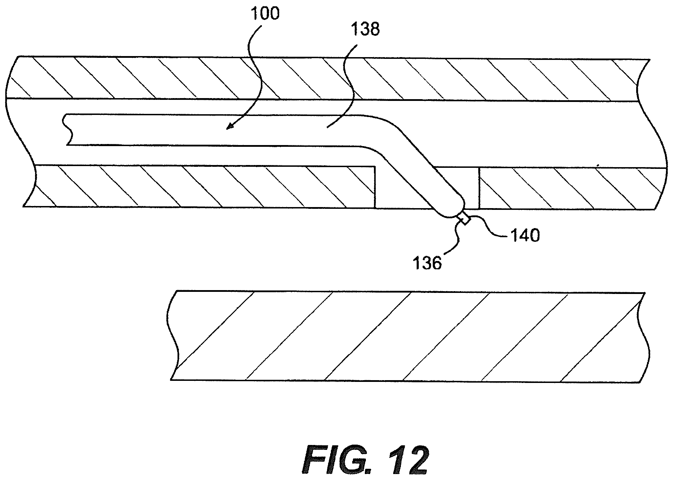

Figure 12 is an enlarged partial cross-sectional view showing a portion of re-entry device 100 and intima 26. With reference to figure 12, it will be appreciated that re-entry device 100 comprises a body 138 and a core 136. In the embodiment of figure 12, core 136 has been advanced so that a portion of core 136 extends beyond body 138. For purposes of illustration and exposition, the portion of core 136 extending beyond body 138 is referred to as a penetrator 140. Embodiments of re-entry device 100 are contemplated in which penetrator 140 is fixed in the position shown in figure 12.

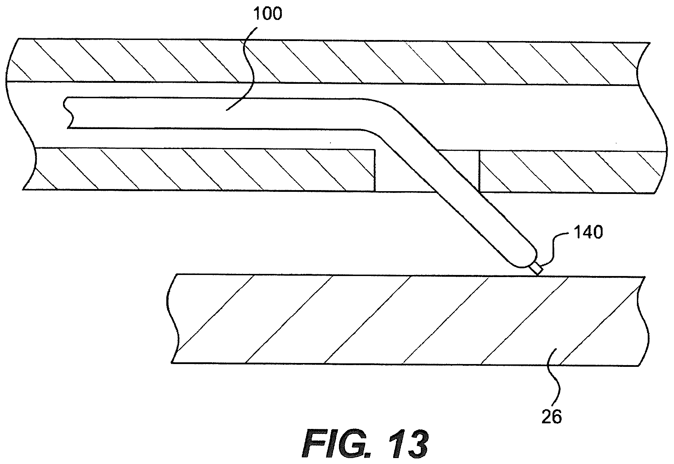

Figure 13 is an enlarged partial cross-sectional view showing a portion of re-entry device 100. In the embodiment of figure 13, re-entry device 100 has been advanced so that penetrator 140 is shown contacting intima 26 in figure 13.

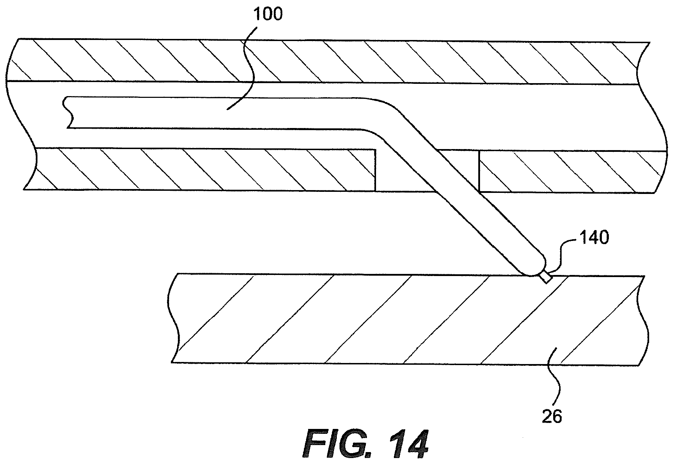

Figure 14 is an enlarged partial cross-sectional view showing a portion of re-entry device 100. In the embodiment of figure 14, penetrator 140 of re-entry device 100 has pierced intima 26.

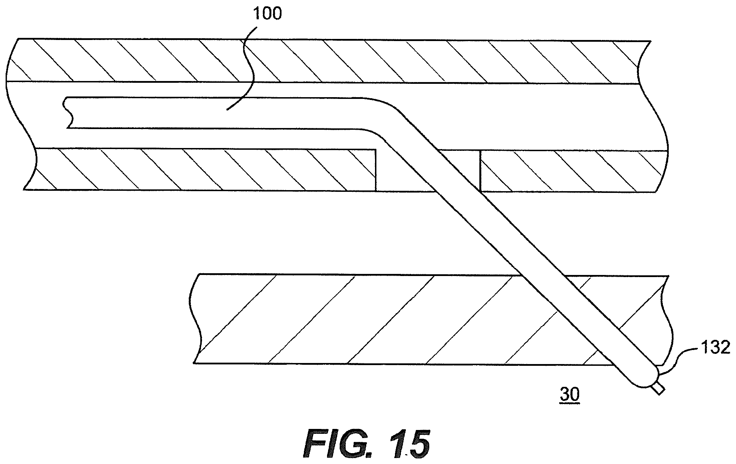

Figure 15 is an enlarged partial cross-sectional view showing a portion of re-entry device 100. In the embodiment of figure 15, re-entry device 100 has been advanced so that distal end 132 of re-entry device 100 is disposed in true lumen 30.

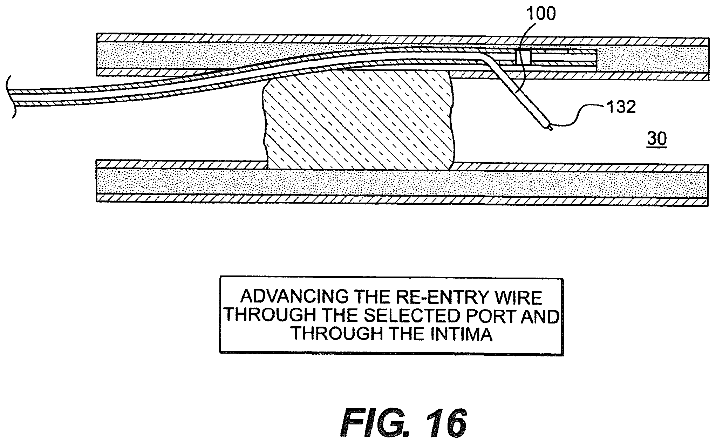

Figure 16 is a partial cross-sectional view of re-entry device 100 shown in the previous figure. Figure 16 has a different scale than the previous figure so that more of the surrounding context is visible in figure 16. In figure 16, distal end 132 of re-entry device 100 can be seen residing in true lumen 30.

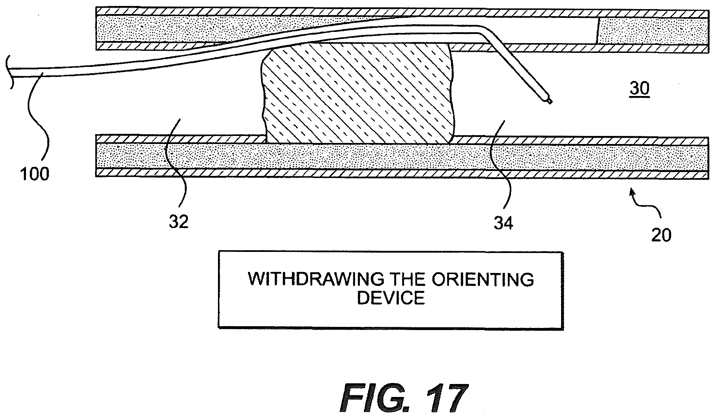

Figure 17 is an additional view of artery 20 shown in the previous figure. In the embodiment of figure 17, orienting device 106 has been withdrawn leaving re-entry device 100 in the position shown in figure 17. Devices such as balloon angioplasty catheters and atherectomy catheters may be advanced over re-entry device 100. In this way, these devices may be used in conjunction with re-entry device 100 to establish a blood flow path between proximal segment 32 of true lumen 30 and distal segment 34 of true lumen 30. This path allows blood to flow around occlusion 36.

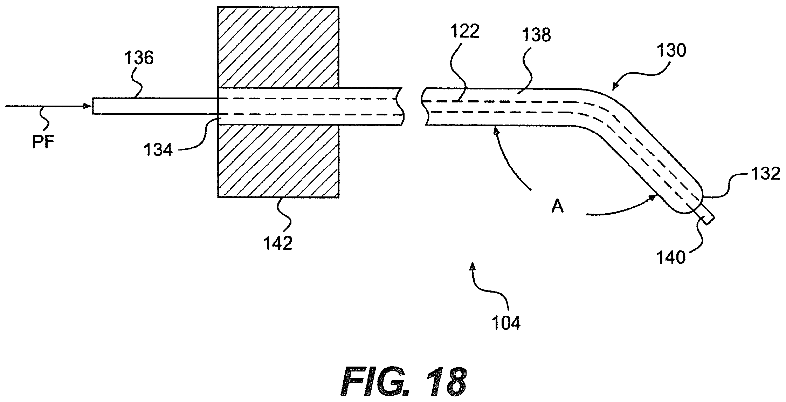

Figure 18 is a plan view of a re-entry device 100 in accordance with the present description. Crossing device 104 includes an elongate body 138 having a distal end 132 and a proximal end 134. In the embodiment of Figure 18, a core 136 extends into a lumen 122 defined by body 138. In some useful embodiments, core 136 is free to advance and retract relative to body 138. When core 136 is moved relative to body 138, penetrator 140 can be caused to selectively assume the retracted position and/or the deployed position. In figure 18, penetrator 140 is shown in the deployed position.

In the embodiment of figure 18, an actuating fixture 142 is fixed to body 138 near proximal end 134. Also in figure 18, a pushing force is shown acting on a proximal portion of core 136. This pushing force is represented by an arrow PF in figure 18. Actuating fixture 142 may be used when creating relative motion between core 136 and body 138. Actuating fixture 142 may be held to hold body 138 relatively stationary and pushing/pulling forces may be applied to core 136 to move core 136 relative to body 138. In figure 18, body 138 of re-entry device 100 is shown being bent at an angle A. Accordingly, it can be said that re-entry device 100 includes a bend 130. In some useful embodiments of re-entry device 100, angle A is between about 120 degrees and about 150 degrees.

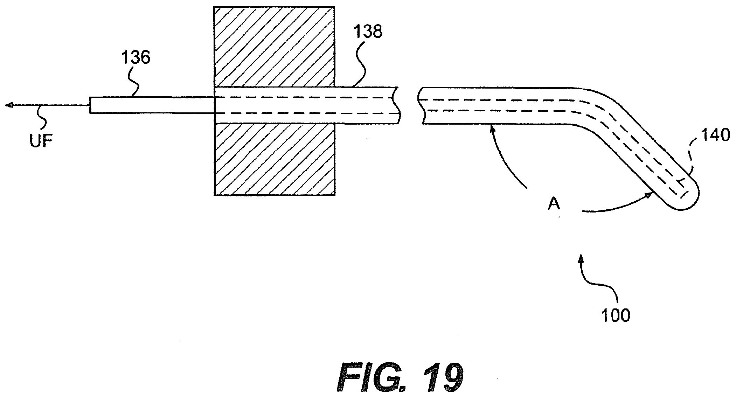

Figure 19 is an additional plan view of re-entry device 100 shown in the previous figure. In the embodiment of figure 19, penetrator 140 is assuming a retracted position. A pulling force applied to core 136 while holding actuating fixture 142 relatively stationary may cause penetrator 140 to assume the retracted position.

With reference to the figures, it will be appreciated that when penetrator 140 is in the retracted position, the distal portion of re-entry device 100 has a less traumatic shape than when penetrator 140 is in the deployed position. Conversely, when penetrator 140 is in the deployed position, the distal portion of re-entry device 100 has a more traumatic shape than when penetrator 140 is in the retracted position.

The position of core 136 may be changed relative to body 138 by apply pushing and/or pulling forces on core 136 and body 138. In figure 19, a pulling force is represented with an arrow UF. A physician may utilize this mechanism to selectively alter the overall shape of a distal portion of re-entry device 100. Changes in the shape of the distal portion of re-entry device 100 may assist in re-entry through the intima.

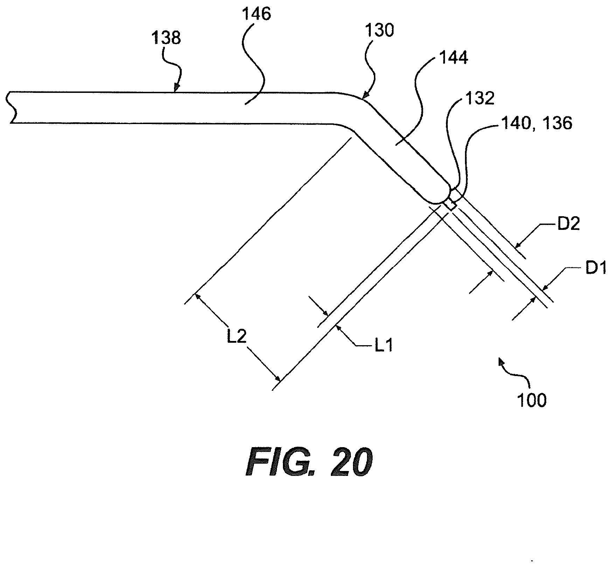

Figure 20 is a plan view of a re-entry device 100 illustrating selected dimensions of re-entry device 100. In the embodiment of Figure 20, a penetrator 140 of core 136 extends beyond a distal end 132 of body 138 by a distance L1. In the embodiment of Figure 20, penetrator 140 has a diameter D1 and body 138 has a diameter D2. With reference to Figure 20, it will be appreciated that diameter D2 of body 138 is greater than diameter D1 of penetrator 140.

With reference to Figure 20, it will be appreciated that body 138 of re-entry device 100 includes a bend 130 near its distal end 132. Body 138 has a distal leg 144 disposed distally of bend 130 and a proximal leg 146 disposed proximally of bend 130. As shown in figure 20, distal leg 144 has a length of L2. With reference to Figure 20, it will be appreciated that length L2 is greater than distance L1.

In some useful embodiments, diameter D1 of penetrator 140 is between about 0.0020 inches and about 0.0055 inches.

In some useful embodiments, diameter D2 of body 138 is between about 0.008 inches and about 0.015 inches.

In some useful embodiments, length L1 of penetrator 140 is between about 0.003 inches and about 0.012 inches.

In some useful embodiments, length L2 of distal leg 144 is between about 0.040 inches and about 0.080 inches.

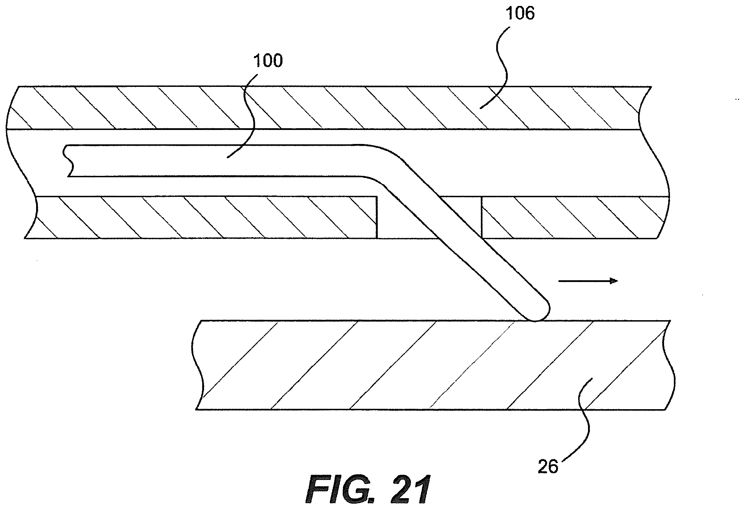

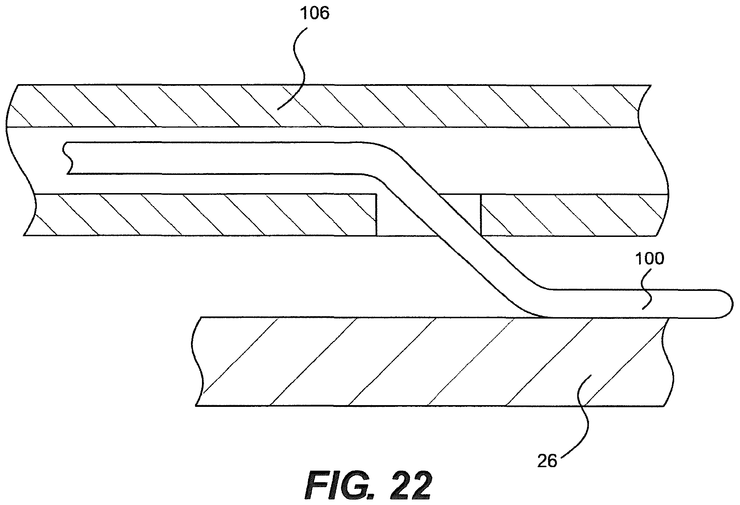

Figure 21 and figure 22 illustrate a method in which re-entry device 100 has been advanced while core 136 is in a retracted position. With reference to the figures, it will be appreciated that re-entry device 100 has not penetrated intima 26. Instead, re-entry device has been advanced between intima 26 and the exterior of orienting device 106.

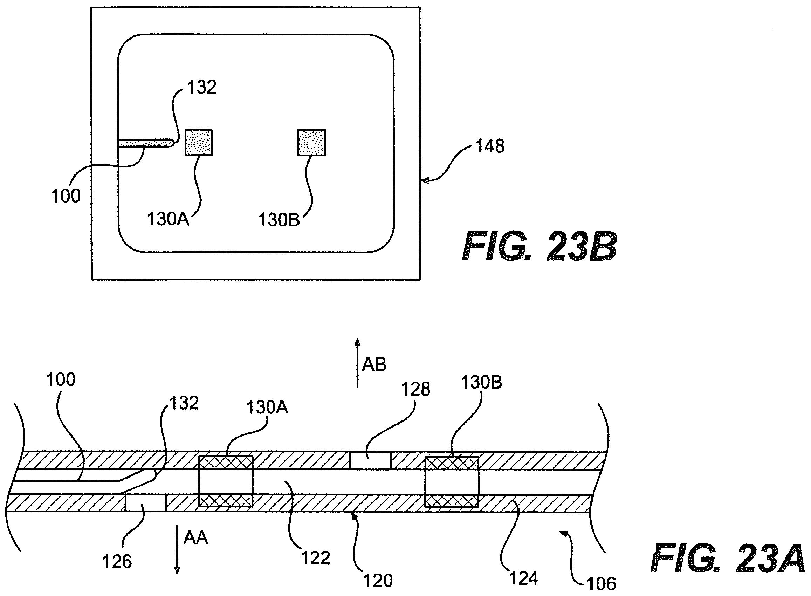

Figure 23A is a cross-sectional view of an orienting device 106. Orienting device 106 includes a shaft 120 comprising a wall 124 defining a lumen 122. Wall 124 defines a first aperture 126 and a second aperture 128 that are both in fluid communication with lumen 122. In the embodiment of Figure 23A, first aperture 126 extends away from lumen 122 in a first direction that is represented by a first arrow AA in Figure 23A. Second aperture 128 extends away from lumen 122 in a second direction that is represented by a second arrow AB in Figure 23A. In Figure 23A, first arrow AA and second arrow AB extend in generally opposite directions. Accordingly, the first direction is about 180 degrees from the second direction.

In the embodiment of Figure 23A, first aperture 126 and second aperture 128 are longitudinally separated from one another. Orienting device 106 includes a first radiopaque marker 130A that is located between first aperture 126 and second aperture 128. A second radiopaque marker 130B of orienting device 106 is located distally of second aperture 128.

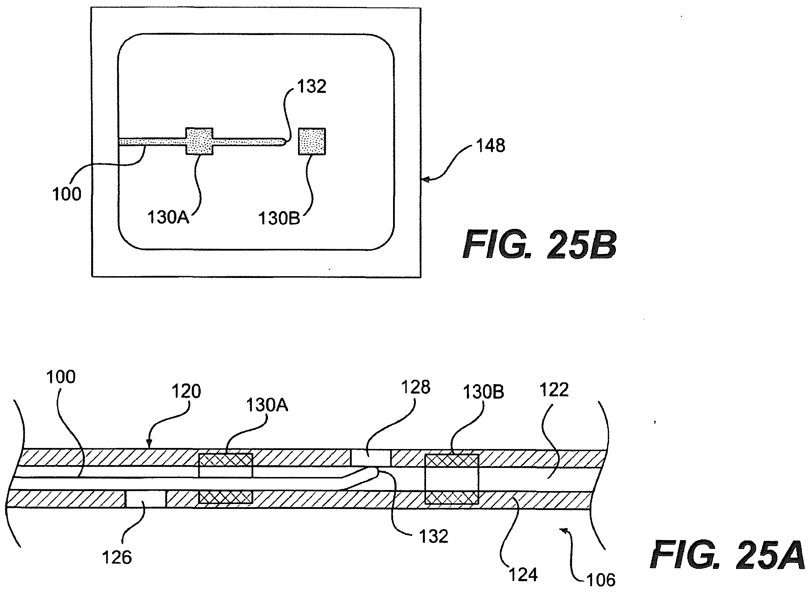

A re-entry device 100 is disposed in lumen 122 of orienting device 106. In the embodiment of Figure 25A, first radiopaque marker 130A, second radiopaque marker 130B and re-entry device 100 comprise radiopaque materials. Because of the radiopaque nature of their materials of construction, first radiopaque marker 130A, second radiopaque marker 130B, and re-entry device 100 will all be visible on a fluoroscopic display during a fluoroscopic procedure.

Figure 23B is a representation of a fluoroscopic display 148. First radiopaque marker 130A, second radiopaque marker 130B, and re-entry device 100 are visible in fluoroscopic display 148. In the embodiment of figure 23B, distal end 132 of re-entry device 100 is located slightly proximal of first radiopaque marker 130A. Accordingly, re-entry device 100 is seen extending across fluoroscopic display 148 and ending just short of first radiopaque marker 130A. When a physician views display 148 shown in figure 23B, the physician may infer that distal end 132 is proximate first aperture 126 of orienting device 106. After determining that distal end 132 of re-entry device 100 is in this location, the physician can rotate re-entry device 100 until distal end 132 enters into first aperture 126.

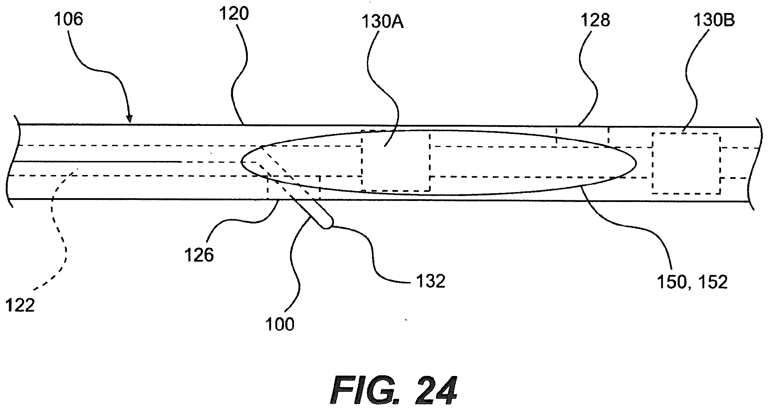

Figure 24 is a plan view including orienting device 106 shown in the previous figure. In figure 24, a distal portion of re-entry device 100 can be seen extending through first aperture 126. First aperture 126 and second aperture 128 both fluidly communicate with lumen 122 of orienting device 106. Orienting device 106 includes a first radiopaque marker 130A that is located between first aperture 126 and second aperture 128. A second radiopaque marker 130B of orienting device 106 is located distally of second aperture 128.

Orienting device 106 comprises an elongate shaft 120, a first orienting element 150, and second orienting element (not visible in figure 24). First orienting element 150 comprises a first balloon 152 and second orienting element comprises a second balloon. When these balloons are inflated between the adventitia and the intima of a blood vessel, orienting device 106 will orient itself within the blood vessel so that either first aperture 126 or second aperture 128 will open toward a true lumen of the artery. The physician may select the aperture opening toward the true lumen using methods described herein. The physician may then use methods in accordance with this disclosure to insert distal end 132 of re-entry device 100 through the selected aperture.

Figure 25A is a cross-sectional view of an orienting device 106. Orienting device 106 includes a shaft 120 comprising a wall 124 defining a lumen 122. In the embodiment of Figure 25, a re-entry device 100 is disposed in lumen 122. Wall 124 defines a first aperture 126 and a second aperture 128 that are both in fluid communication with lumen 122.

In the embodiment of Figure 25A, first aperture 126 and second aperture 128 are longitudinally separated from one another. Orienting device 106 includes a first radiopaque marker 130A that is located between first aperture 126 and second aperture 128. Orienting device 106 also comprises a second radiopaque marker 130B that is located distally of second aperture 128. With reference to figure 25A, it will be appreciated that first radiopaque marker 130A and second radiopaque maker 130B are both surrounded by wall 124 of shaft 120.

In the embodiment of Figure 25A, first radiopaque marker 130A, second radiopaque marker 130B and re-entry device 100 comprise radiopaque materials. Because of the radiopaque nature of their materials of construction, first radiopaque marker 130A, second radiopaque marker 130B, and re-entry device 100 will all be visible on a fluoroscopic display during a fluoroscopic procedure.

Figure 25B is a representation of a fluoroscopic display 148. First radiopaque marker 130A, second radiopaque marker 130B, and re-entry device 100 are visible in fluoroscopic display 148. In the embodiment of figure 25B, distal end 132 of re-entry device 100 is located slightly proximal of second radiopaque marker 130B. Accordingly, re-entry device 100 is seen extending across fluoroscopic display 148 and ending just short of second radiopaque marker 130B. When a physician views display 148 shown in figure 25B, the physician may infer that distal end 132 is proximate second aperture 126 of orienting device 106. After determining that distal end 132 of re-entry device 100 is in this location, the physician can rotate re-entry device 100 until distal end 132 enters into second aperture 126.

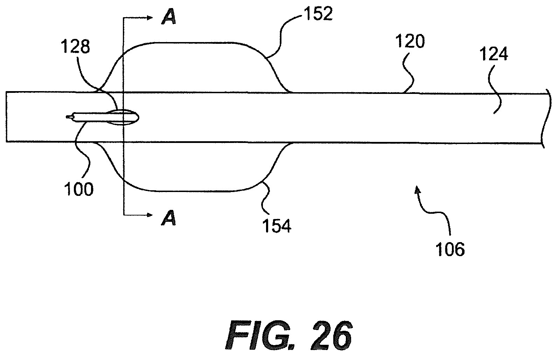

Figure 26 is a plan view including orienting device 106 shown in the previous figure. Orienting device 106 comprises an elongate shaft 120, a first balloon 152, and a second balloon 154. In the embodiment of Figure 26, first balloon 152 and second balloon 154 are both formed from extruded portions of an outer wall 124 of elongate shaft 120. Outer wall 124 defines a second aperture 128. In Figure 26, a re-entry device 100 is shown extending through second aperture 128.

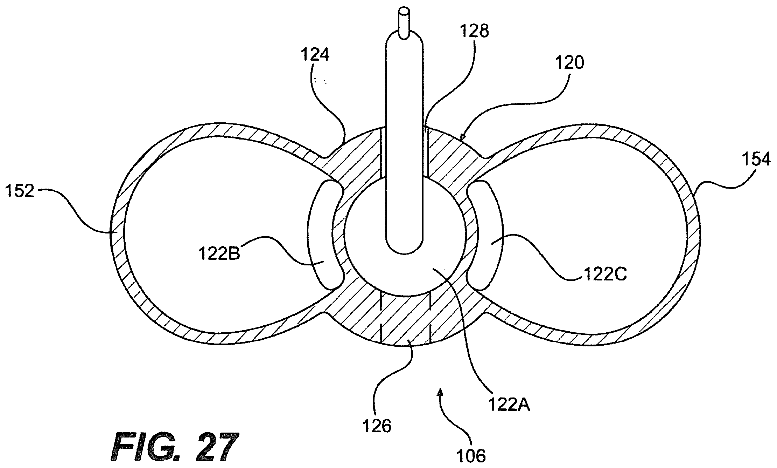

Figure 27 is a cross sectional view of orienting device 106 taken along line A-A shown in Figure 26. With reference to Figure 27, it will be appreciated that elongate shaft 120 defines a lumen 122A, a first planetary lumen 122B, and a second planetary lumen 122C. The planetary lumens are defined in part by an outer wall 124 of elongate shaft 120. Outer wall 124 defines a first aperture 126 and a second aperture 128.

In the embodiment of Figure 27, a first balloon 152 is formed of an extruded portion of outer wall 124 of elongate shaft 120. First balloon 152 defines an interior that is in fluid communication with first planetary lumen 122B. In the embodiment of Figure 27, first balloon 152 and elongate shaft 120 are monolithic. As shown in Figure 27, first balloon 152 and outer wall 124 of elongate shaft 120 are seamlessly formed from a single piece of material. With reference to Figure 27, it will be appreciated that second balloon 154 defines an interior that is in fluid communication with second planetary lumen 122C. In the embodiment of Figure 27, second balloon 154 comprises an extruded portion of outer wall 124 of elongate shaft 120.

As shown in Figure 27, second balloon 154 and elongate shaft 120 are seamlessly formed from a single piece of material. Second balloon 154 may be formed, for example, by extruding a portion of outer wall 124 . In some useful embodiments, elongate shaft 120 comprises a thermoplastic material. When this is the case, elongate shaft 120 may be formed, for example, using an extrusion process. Also when this is the case, first balloon 152 and second balloon 154 may be formed by further extruding outer wall 124 of elongate shaft 120.

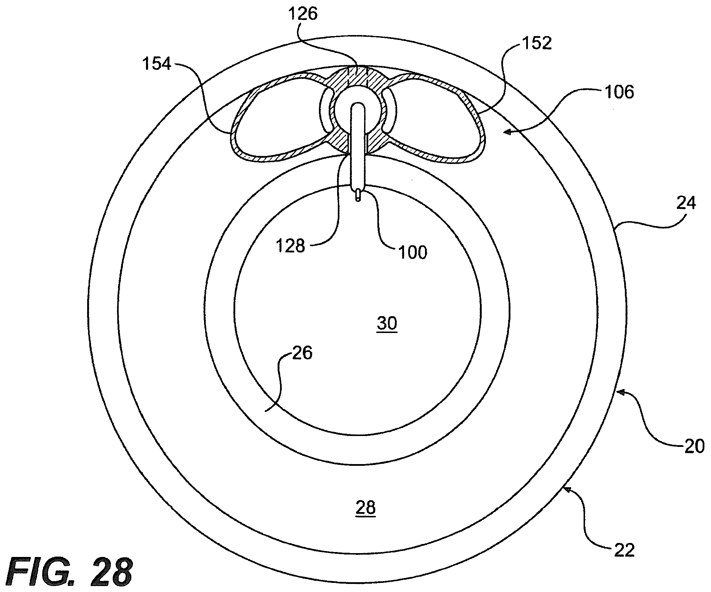

Figure 28 is a cross-sectional view of an artery 20 having a wall 22. In figure 28, wall 22 of artery 20 is shown having three layers. The outermost layer of wall 22 is the adventitia 24 and the innermost layer of wall 22 is the intima 26. The tissues extending between intima 26 and adventitia 24 may be collectively referred to as the media 28. For purposes of illustration, intima 26, media 28 and adventitia 24 are each shown as a single homogenous layer in figure 28. In the human body, however, the intima and the media each comprise a number of sublayers. The transition between the external most portion of the intima and the internal most portion of the media is sometimes referred to as the subintimal space. Intima 26 defines a true lumen 30 of artery 20.

In figure 28, orienting device 106 is shown disposed between adventitia 24 and intima 26 of artery 20. Orienting device 106 may be used to direct a re-entry device 100 toward true lumen 30 of artery 20 as shown in figure 28. The first aperture 126 and second aperture 128 are generally oriented at a right angle to a plane defined by first balloon 152 and second balloon 154. With this arrangement, each aperture is either directed toward true lumen 30 of artery 20 or 180 degrees away from true lumen 30 when first balloon 152 and second balloon 154 are inflated. In this way, orienting device 106 reduces the number of directions an aperture may be facing from 360 degrees of freedom to two degrees of freedom, 180 degrees apart.

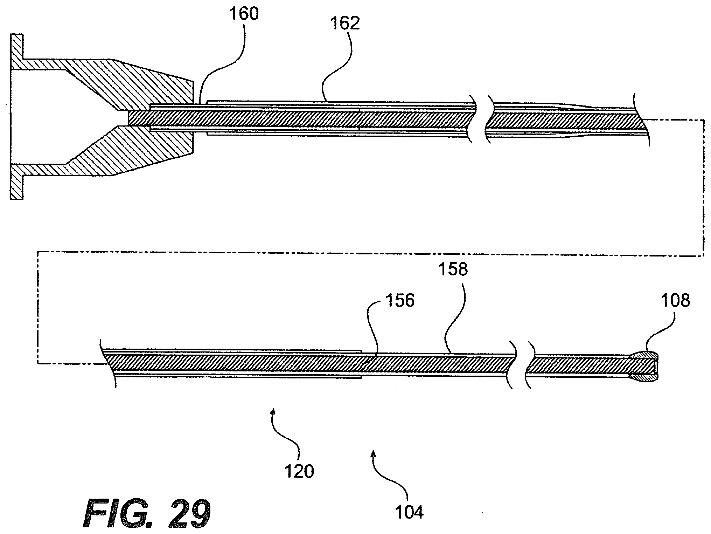

Figure 29 is a partial cross-sectional view of an exemplary crossing device 104. Crossing device 104 of Figure 29 comprises a tip 108 that is fixed to a distal end of a shaft 120. In the exemplary embodiment of Figure 29, shaft 120 comprises a coil 156, a sleeve 158, a tubular body 160, and a sheath 162.

Tip 108 is fixed to a distal portion of coil 156. Coil 156 comprises a plurality of filars that are wound in a generally helical shape. In some useful embodiments of crossing device 104, coil 156 comprises eight, nine or ten filars wound into the shape illustrated in Figure 29. Crossing device 104 includes a sleeve 158 that is disposed about a portion of coil 156. Sleeve 158 may comprise, for example, PET shrink tubing, i.e. polyethylene terephthalate.

Sleeve 158 and coil 156 both extend into a lumen defined by a tubular body 160. Tubular body 160 may comprise, for example hypodermic tubing formed of Nitnol, i.e. nickel titanium. With reference to Figure 29, it will be appreciated that a proximal portion of sleeve 158 is disposed between tubular body 160 and coil 156. In some embodiments of crossing device 104, a distal portion of tubular body 160 defines a helical cut. This helical cut may be formed, for example, using a laser cutting process. The helical cut may be shaped and dimensioned to provide an advantageous transition in lateral stiffness proximate the distal end of tubular body 160.

A proximal portion of coil 156 extends proximally beyond the distal end of tubular body 160. A hub is fixed to a proximal portion of coil 156 and a proximal portion of tubular body 160. The hub may comprise, for example, a luer fitting. A sheath 162 is disposed about a portion of tubular body 160 and a portion of sleeve 158. In some embodiments of crossing device 104, sheath 162 comprises HYTREL, a thermoplastic elastomer.

With reference to Figure 29, it will be appreciated that tubular body 160, coil 156, sleeve 158, and sheath 162 each have a proximal end and a distal end. The proximal end of outer sleeve 158 is disposed between the proximal end of tubular body 160 and the proximal end of sleeve 158. The distal end of sleeve 158 is positioned proximate tip 108 that is fixed to the distal end of coil 156. The distal end of sheath 162 is located between the distal end of tubular body 160 and the distal end of sleeve 158. With reference to Figure 29, it will be appreciated that sheath 162 overlays the distal end of tubular body 160.

With reference to Figure 29, it will be appreciate that tip 108 has a generally rounded shape. The generally rounded shape of tip 108 may reduce the likelihood that crossing device 104 will penetrate the adventitia of an artery. Tip 108 may be formed from a suitable metallic material including but not limited to stainless steel, silver solder, and braze. Tip 108 may also be formed from suitable polymeric materials or adhesives including but not limited to polycarbonate, polyethylene and epoxy. In some embodiments of crossing device 104, the outer surface of tip 108 comprises a generally non-abrasive surface. For example, the outer surface of tip 108 may have a surface roughness of about 25 micrometers or less. A tip member having a relatively smooth outer surface may reduce the likelihood that the tip member will abrade the adventitia of an artery.

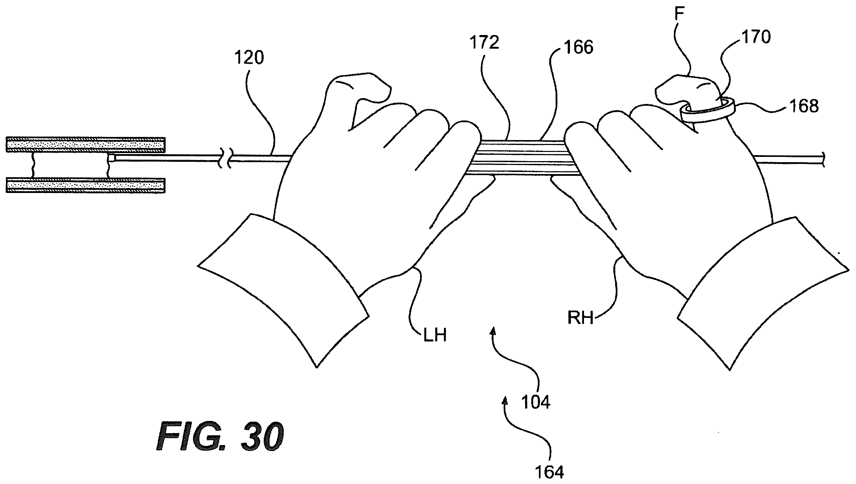

Figure 30 is a plan view showing an assembly 164 including crossing device 104 shown in the previous figure. In the embodiment of Figure 30, a drive assembly 166 is coupled to crossing device 104. In Figure 30, drive assembly 166 is shown disposed about a proximal portion of shaft 120 of crossing device 104. Drive assembly 166 comprises a handle body 172 and an anchor 168.

As shown in Figure 30, handle body 172 of drive assembly 166 is long enough to receive the thumb and forefingers of a right hand RH and a left hand LH. Anchor 168 of drive assembly 166 defines a hole 170. With reference to Figure 30, it will be appreciated that a finger F of right hand RH is extending through hole 170 in anchor 168. Left hand LH and right hand RH may rotate handle body 172 of drive assembly 166. When this is the case, finger F extending through anchor 168 prevents anchor 168 from rotating while handle body 172 rotates.

In Figure 30, a distal portion of handle body 172 is positioned between the thumb and forefinger of a left hand LH. A proximal portion of handle body 172 is disposed between the thumb and forefinger of a right hand RH. In some useful methods, crossing device 104 is rotated and axially advanced simultaneously. Rotation of crossing device 104 can be achieved by rolling handle body 172 between the thumb and forefinger one hand. Two hands can also be used as shown in Figure 30. Rotating crossing device 104 assures that the coefficient of friction at the interface between the crossing device and the surrounding tissue will be a kinetic coefficient of friction and not a static coefficient of friction.

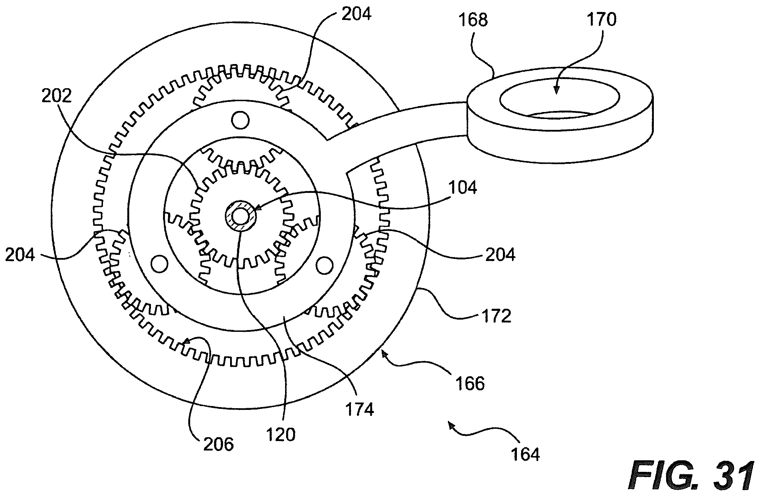

Figure 31 is a cross-sectional view of assembly 164 shown in the previous figure. Assembly 164 includes a drive assembly 166 and a crossing device 104. With reference to figure 31, it will be appreciated that drive assembly 166 includes a central gear 202 that is fixed to shaft 120 of crossing device 104. Drive assembly 166 also includes a handle body 172. An internal gear 206 is fixed to handle body 172.

A plurality of planetary gears 204 are disposed between central gear 202. A ring 174 maintains the spacing between adjacent pairs planetary gears 204. Anchor 168 is fixed to ring 174. Anchor 168 defines a hole 170. Central gear 202, planetary gears 204, and internal gear 206 together form a gear train providing a mechanical advantage. Due to this mechanical advantage, a single rotation of handle body 172 results in many rotations of shaft 120 of crossing device 104.

In some useful methods in accordance with the present disclosure, crossing device 104 is rotated at a rotational speed of between about 2 revolutions per minute and about 200 revolutions per minute. In some particularly useful methods in accordance with the present disclosure, crossing device 104 is rotated at a rotational speed of between about 50 revolutions per minute and about 150 revolutions per minute. Crossing device 104 may be rotated by hand as depicted in the previous figure. It is also contemplated that a mechanical device (e.g., an electric motor) may be used to rotate crossing device 104.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

Further embodiments of the present invention are described by the following aspects:
1. A method of facilitating treatment via a vascular wall defining a vascular lumen containing an occlusion therein, the method comprising:
   providing a first intravascular device having a distal portion and at least one aperture;
   positioning the distal portion of the first intravascular device in the vascular wall;
   providing a reentry device having a body and a distal tip, the distal tip having a natural state and a compressed state;
   inserting the distal tip, in the compressed state, in the distal portion of the first intravascular device; and
   advancing the distal tip, in the natural state, through the at least one aperture of the first intravascular device.
2. The method of aspect 1, wherein when the distal tip is in the natural state, the distal tip forms an angle with the body.
3. The method of aspect 2, wherein the angle is between about 120 degrees and about 150 degrees.
4. The method of aspect 1, further including directing the distal tip of the reentry device towards the vascular lumen and into contact with an intimal layer of the vascular wall.
5. The method of aspect 1, wherein the reentry device includes a body, the body defining a device lumen, and a core having a distal tip, and wherein the core is located in the device lumen and is movable relative to the device lumen.
6. The method of aspect 5, further including moving the distal tip of the core of the reentry device beyond the distal tip of the reentry device.
7. The method of aspect 5, further comprising piercing an intimal layer of the vascular wall with the core of the reentry device, and advancing the distal tip of the reentry device into the vascular lumen.
8. The method of aspect 7, wherein the distal tip of the core includes a penetrator, and wherein the penetrator is configured to pierce the vascular wall.
9. The method of aspect 1, wherein the at least one aperture includes two apertures, and wherein the first aperture is located proximal the second aperture.
10. The method of aspect 9, wherein the first intravascular device includes at least one radiopaque marker located distal the first aperture.
11. The method of aspect 10, wherein the at least one radiopaque marker comprises a first radiopaque marker located between the first aperture and the second aperture, and a second radiopaque marker located distal the second aperture.
12. The method of aspect 11, further including providing a flouroscopic display and positioning the distal tip of the reentry device proximal the first radiopaque marker of the first intravascular device.
13. The method of aspect 12, further including rotating the reentry device until the distal tip of the reentry device enters the first aperture of the first intravascular device.
14. The method of aspect 11, further including providing a flouroscopic display and positioning the distal tip of the reentry device proximal the second radiopaque marker of the first intravascular device and distal the first radiopaque marker of the first intravascular device.
15. The method of aspect 14, further including rotating the reentry device until the distal tip of the reentry device enters the second aperture of the first intravascular device.
16. The method of aspect 1, further including:
   providing a second intravascular device having a distal portion;
   positioning the second intravascular device in the vascular wall prior to positioning the distal portion of the first intravascular device in the vascular wall; and
   advancing the distal portion of the second intravascular device in the vascular wall beyond the occlusion.
17. The method of aspect 16, further including advancing a guidewire through a lumen of the second intravascular device, and removing the second intravascular device from the vascular wall while leaving the guidewire in the vascular wall.
18. The method of aspect 17, wherein positioning the distal portion of the first intravascular device in the vascular wall comprises advancing the first intravascular device over the guidewire.
19. The method of aspect 18, further comprising withdrawing the guidewire from a guidewire lumen of the first intravascular device.
20. The method of aspect 19, further comprising advancing at least a portion of the reentry device into the guidewire lumen of the first intravascular device.
21. The method of aspect 20, further comprising removing the first intravascular device from the vascular wall while a distal portion of the reentry device is extending through an intimal portion of the vascular wall.
22. The method of aspect 21, further comprising advancing a therapy catheter over the reentry device.
23. A method of facilitating treatment via a vascular wall defining a vascular lumen containing an occlusion therein, the method comprising:
   providing a first intravascular device having a distal portion and at least one aperture;
   inserting the first intravascular device into the vascular lumen;
   positioning the distal portion of the first intravascular device in the vascular wall;
   providing a second intravascular device having a body, a core, and a distal tip, the core being movable relative to the body;
   inserting the distal tip of the second intravascular device in the distal portion of the first intravascular device; and
   advancing the distal tip of the second intravascular device through at least one aperture of the first intravascular device.
24. The method of aspect 23, further including positioning the at least one aperture of the first intravascular device beyond the occlusion.
25. The method of aspect 23, further including positioning the distal tip of the second intravascular device in the aperture of the first intravascular device.
26. The method of aspect 25, wherein positioning the distal tip of the second intravascular device includes rotating the second intravascular device.
27. The method of aspect 23, wherein the at least one aperture includes two apertures, and wherein the first aperture is located proximal the second aperture.
28. The method of aspect 27, further including positioning the distal tip of the second intravascular device in the second aperture of the first intravascular device.
29. The method of aspect 27, further comprising orienting the at least one aperture of the first intravascular device towards the vascular lumen and orienting the second aperture towards an adventitia of the vascular wall.
30. The method of aspect 27, wherein the at least one aperture includes two apertures, and wherein the first aperture is located proximal the second aperture.
31. The method of aspect 30, further including:
   advancing a distal tip of a second intravascular device through one of the first aperture and the second aperture; and
   determining whether the distal tip of the second intravascular device is directed toward the vascular lumen or directed toward the adventitia of the vascular wall.
32. The method of aspect 31, wherein determining whether the distal tip of the second intravascular device is directed toward the vascular lumen or directed toward the adventitia comprises viewing the distal tip of the second intravascular device using fluoroscopy.
33. The method of aspect 31, further comprising advancing the distal tip of the second intravascular device through an intimal portion of the vascular wall if it is determined that the distal tip of the second intravascular device is directed toward the vascular lumen.
34. The method of aspect 31, further comprising advancing the distal tip of the second intravascular device into the vascular lumen if it is determined that the distal tip of the second intravascular device is directed toward the vascular lumen.
35. The method of aspect 31, further comprising withdrawing the distal tip of the second intravascular device into the lumen of the first intravascular device if it is determined that the distal tip of the second intravascular device is directed toward the adventitia of the vascular wall.
36. The method of aspect 35, further comprising advancing the distal tip of the second intravascular device through the other of the first aperture and the second aperture.
37. The method of aspect 36, further comprising determining whether the distal tip of the second intravascular device is directed toward the vascular lumen or directed toward the adventitia of the vascular wall.
38. The method of aspect 27, wherein the first intravascular device includes at least one radiopaque marker located distal the first aperture.
39. The method of aspect 38, wherein the at least one radiopaque marker comprises a first radiopaque marker located between the first aperture and the second aperture, and a second radiopaque marker located distal the second aperture.
40. The method of aspect 39, further including providing a flouroscopic display and positioning the distal tip of the second intravascular device proximal the first radiopaque marker of the first intravascular device.
41. The method of aspect 40, further including rotating the second intravascular device until the distal tip of the second intravascular device enters the first aperture of the first intravascular device.
42. The method of aspect 39, further including providing a flouroscopic display and positioning the distal tip of the second intravascular device proximal the second radiopaque marker of the first intravascular device and distal the first radiopaque marker of the first intravascular device.
43. The method of aspect 42, further including rotating the second intravascular device until the distal tip of the second intravascular device enters the second aperture of the first intravascular device.
44. The method of aspect 23, further including advancing the distal tip of second intravascular device into the vascular lumen.
45. The method of aspect 23, wherein the body of the second intravascular device defines a device lumen, the core of the second intravascular device includes a distal tip, and wherein the core is located in the device lumen.
46. The method of aspect 45, further including moving the distal tip of the core beyond the distal tip of the second intravascular device.
47. The method of aspect 46, further including advancing the distal tip of the second intravascular device into the vascular lumen by piercing an intimal layer of the vascular wall with the core of the second intravascular device.
48. The method of aspect 47, wherein the distal tip of the core includes a penetrator, and wherein the penetrator is configured to pierce the vascular wall.
49. The method of aspect 45, wherein when the distal tip of the core is within the device lumen, the distal tip of the second intravascular device is blunt.
50. The method of aspect 49, wherein when the distal tip of the core is advanced beyond the device lumen, the distal tip of the second intravascular device is sharp.
51. The method of aspect 23, further including removing the first intravascular device from the vascular wall while leaving the second intravascular device in the vascular wall.
52. The method of aspect 23, further including:
   providing a third intravascular device having a distal portion;
   positioning the third intravascular device in the vascular wall prior to positioning the distal portion of the first intravascular device in the vascular wall; and
   advancing the distal portion of the third intravascular device in the vascular wall beyond the occlusion.
53. The method of aspect 52, further including advancing a guidewire through a lumen of the third intravascular device, and removing the third intravascular device from the vascular wall while leaving the guidewire in the vascular wall.
54. The method of aspect 23, wherein the first intravascular device further includes at least one orienting element for orienting the at least one aperture of the first intravascular device towards the vascular wall.
55. The method of aspect 54, wherein the at least one orienting element comprises a balloon, and orienting the at least one aperture of the first intravascular device towards the vascular wall includes inflating the balloon.
56. The method of aspect 55, wherein the at least one orienting element comprises two balloons, and orienting the at least one aperture of the first intravascular device towards the vascular wall includes inflating the two balloons.
57. A method of facilitating treatment via a vascular wall defining a vascular lumen containing an occlusion therein, the method comprising:
   providing an orienting device having a distal portion and at least one aperture;
   positioning the distal portion of the first intravascular device in the vascular wall;
   orienting the first intravascular device so that the at least one aperture faces an intimal layer of the vascular wall;
   providing a reentry device having a body and a penetrator extending distally beyond a distal end of the body; and
   advancing the penetrator and the distal end of the body through the at least one aperture of the first intravascular device.
58. The method of aspect 57, further comprising contacting the intimal layer of the vascular wall with the penetrator.
59. The method of aspect 58, further comprising contacting the intimal layer of the vascular wall with the distal end of the body.
60. The method of aspect 59, wherein the penetrator contacts the intimal layer of the vascular wall before the distal end of the body contacts the intimal layer of the vascular wall.
61. The method of aspect 57, further comprising piercing the intimal layer of the vascular wall with the penetrator.
62. The method of aspect 61, further comprising piercing the intimal layer of the vascular wall with the distal end of the body.
63. The method of aspect 62, wherein the penetrator pierces the intimal layer of the vascular wall before the distal end of the body pierces the intimal layer of the vascular wall.
64. The method of aspect 57, wherein providing the reentry device comprises providing a reentry device having a core including the penetrator, the core being movable relative to the body of the reentry device, and further including moving the core relative to the body.
65. The method of aspect 64, wherein moving the core relative to the body comprises moving a distal end of the core beyond the distal end of the body.
66. The method of aspect 57, wherein providing the reentry device comprises providing a reentry device in which the penetrator is fixed relative to the body.
67. The method of aspect 57, further comprising advancing the distal end of the body of the reentry device into the vascular lumen.
68. A method of facilitating treatment via a vascular wall defining a vascular lumen containing an occlusion therein, the method comprising:
   providing a first intravascular device having a distal portion and at least one aperture;
   positioning the distal portion of the first intravascular device in the vascular wall;
   orienting the first intravascular device so that the at least one aperture faces an intimal portion of the vascular wall;
   providing a reentry device having a first configuration in which a distal tip of the reentry device is sharp and a second configuration in which the distal tip of the reentry device is blunt;
   advancing the distal tip of the reentry device through the at least one aperture of the first intravascular device; and
   advancing the distal tip of the reentry device through the intimal portion of the vascular wall.
69. The method of aspect 68, wherein the distal tip of the reentry device is advanced through the intimal portion of the vascular wall while the reentry device is in the first configuration.
70. The method of aspect 69, further comprising causing the reentry device to assume the second configuration after the distal tip has advanced through the intimal portion of the vascular wall.
71. The method of aspect 69, further comprising advancing the distal tip of the reentry device into the vascular lumen.
72. The method of aspect 71, further comprising causing the reentry device to assume the second configuration while the distal tip of the reentry device is disposed in the vascular lumen.
73. The method of aspect 68, wherein:
   the reentry device comprises a body defining a device lumen and a core disposed in the device lumen; and
   the method comprises moving the core in a distal direction relative to the body to cause the reentry device to assume the first configuration.
74. The method of aspect 68, wherein:
   the reentry device comprises a body defining a device lumen and a core disposed in the device lumen; and
   the method comprises moving the core in a proximal direction relative to the body to cause the reentry device to assume the second configuration.
75. The method of aspect 68, wherein:
   the reentry device comprises a body defining a device lumen and a core disposed in the device lumen; and
   a distal portion of the core extends distally beyond the device lumen when the reentry device is in the first configuration.
76. The method of aspect 68, wherein:
   the reentry device comprises a body defining a device lumen and a core disposed in the device lumen; and
   a distal portion of the core is disposed within the device lumen when the reentry device is in the second configuration.
77. An apparatus for facilitating treatment via a vascular wall defining a vascular lumen containing an occlusion therein, the apparatus comprising:
   a first intravascular device having a distal portion, the distal portion including at least one aperture, at least one radiopaque marker, and at least one orienting element; and
   a reentry device having a body and a distal tip, the distal tip having a natural state and a compressed state.
78. The apparatus of aspect 79, wherein when the reentry device is in the natural state, the distal tip forms an angle with the body.
79. The apparatus of aspect 78, wherein the angle is between about 120 degrees and about 150 degrees.
80. The apparatus of aspect 77, wherein when the reentry device is in the compressed state, the distal tip is substantially aligned with the body.
81. The apparatus of aspect 77, wherein the reentry device includes a body, the body defining a device lumen, and a core having a distal tip, and wherein the core is located in the device lumen and is movable relative to the device lumen.
82. The apparatus of aspect 81, wherein the core of the reentry device is configured to move beyond the distal tip of the reentry device.
83. The apparatus of claim 81, wherein the core of the reentry device is configured to pierce the vascular wall.
84. An apparatus for facilitating treatment via a vascular wall defining a vascular lumen containing an occlusion therein, the apparatus comprising:
   a first intravascular device having a distal portion, at least one aperture, and at least one orienting element; and
   a second intravascular device having a distal tip, a body, and a core movable relative to the body.
85. The apparatus of aspect 84, wherein the at least one aperture includes two apertures, and wherein the first aperture is located proximal the second aperture.
86. The apparatus of aspect 84, further including at least one radiopaque marker located distal the first aperture.
87. The apparatus of aspect 86, wherein the at least one radiopaque marker comprises a first radiopaque marker located between the first aperture and the second aperture, and a second radiopaque marker located distal the second aperture.
88. The apparatus of aspect 84, wherein the body of the second intravascular device defines a device lumen, the core of the second intravascular device includes a distal tip, and wherein the core is located in the device lumen.
89. The apparatus of aspect 88, wherein the distal tip of the core of the second intravascular device is configured to move beyond the distal tip of the second intravascular device.
90. The apparatus of aspect 88, wherein the core of the second intravascular device is configured to pierce the vascular wall.
91. The apparatus of aspect 88, wherein the core includes a penetrator and the penetrator is configured to pierce the vascular wall.
92. The apparatus of aspect 88, wherein the second intravascular device includes a first configuration in which the distal tip of the second intravascular device is sharp and a second configuration in which the distal tip of the second intravascular device is blunt.
93. The apparatus of aspect 88, wherein when the distal tip of the core is within the device lumen, the distal tip of the second intravascular device is blunt.
94. The apparatus of aspect 88, wherein when the distal tip of the core is advanced beyond the device lumen, the distal tip of the second intravascular device is sharp.
95. The apparatus of aspect 84, wherein the at least one orienting element comprises an inflatable member configured to orient the first intravascular device after being inflated.
96. The apparatus of aspect 95, wherein the at least one orienting element comprises two inflatable members, configured to orient the first intravascular device after being inflated.
97. The apparatus of aspect 96, wherein the first inflatable member extends from a shaft of the first intravascular device in a first direction, the second inflatable member extends from the shaft in a second direction, and the second direction is substantially opposite the first direction.
98. The apparatus of aspect 97, wherein the at least one aperture includes two apertures, and wherein the first aperture is located proximal the second aperture.
99. The apparatus of aspect 98, wherein the first aperture extends in a third direction through a wall of the first intravascular device, and the second aperture extends in a fourth direction through a wall of the first intravascular device.
100. The apparatus of aspect 99, wherein the fourth direction is generally opposite the third direction.
101. The apparatus of aspect 99, wherein the fourth direction is generally perpendicular to the first direction and the second direction.
102. The apparatus of aspect 84, wherein the at least one aperture comprises a first aperture and a second aperture that are longitudinally offset from one another.
103. An apparatus for facilitating treatment via a vascular wall defining a vascular lumen containing an occlusion therein, the apparatus comprising:
   an intravascular device including:
      a shaft having a distal end and a proximal end, the shaft including:
         a coil extending from the distal end of the shaft to the proximal end of the shaft,
         a sleeve, having a proximal end and a distal end, the sleeve extending from the distal end of the shaft and covering a portion of the coil; and
      a tip fixed to the distal end of the shaft; and
   a drive assembly including a handle body and an anchor.
104. The apparatus of aspect 103, wherein the handle body of the drive assembly is rotatable relative to the anchor of the drive assembly.
105. The apparatus of aspect 104, wherein the drive assembly includes a drive mechanism operatively coupling the handle body to the shaft of the intravascular device so that rotation of the handle body rotates the intravascular device.
106. The apparatus of aspect 104, wherein a single rotation of the handle body results in multiple rotations of the shaft of the intravascular device.
107. The apparatus of aspect 104, wherein the drive mechanism comprises a gear train.
108. The apparatus of aspect 104, wherein the drive mechanism comprises a first gear fixed to the shaft of the intravascular device.
109. The apparatus of aspect 108, wherein the first gear is operatively coupled to the handle body via at least one second gear.
110. The apparatus of aspect 109, wherein the at least one second gear comprises teeth that are intermeshed with teeth of the first gear.
111. The apparatus of aspect 110, wherein the teeth of the at least one second gear are intermeshed with teeth of a third gear.
112. The apparatus of aspect 111, wherein the third gear is an internal gear of the handle body.
113. The apparatus of aspect 110, wherein a ring is fixed to the at least one second gear.
114. The apparatus of aspect 113, wherein the anchor is fixed to the ring.
115. The apparatus of aspect 114, wherein the anchor includes a hole configured to receive a finger.
116. The apparatus of aspect 103, wherein the coil includes a plurality of filars wound in a helical shape.
117. An apparatus for facilitating treatment via a vascular wall defining a vascular lumen containing an occlusion therein, the apparatus comprising:
   a reentry device having a first configuration in which a distal tip of the reentry device is sharp and a second configuration in which the distal tip of the reentry device is blunt.
118. The apparatus of aspect 117, wherein the reentry device comprises a body defining a device lumen and a core disposed in the device lumen.
119. The apparatus of aspect 118, wherein a distal portion of the core extends distally beyond the device lumen when the reentry device is assuming the first configuration.
120. The apparatus of aspect 118, wherein a distal portion of the core is disposed within the device lumen when the reentry device is assuming the second configuration.
121. The apparatus of aspect 118, wherein:
   a distal portion of the core has a first lateral extent;
   a distal portion of the body has a second lateral extent; and
   the second lateral extent is greater than the first lateral extent.
122. The apparatus of aspect 117, further comprising an orienting device defining a lumen and at least one aperture fluidly communicating with the lumen.
123. The apparatus of aspect 122, wherein the reentry device is at least partially disposed in the lumen of the orienting device.
124. The apparatus of aspect 122, wherein the reentry device extends through the at least one aperture of the orienting device.
125. The apparatus of aspect 122, wherein:
   the at least one aperture comprises a first aperture and a second aperture; and
   wherein the first aperture and the second aperture are opposite one another so that when the device is disposed in a vascular wall with one aperture facing an adventitia of the vascular wall, the other aperture faces the vascular lumen.
126. The apparatus of aspect 122, wherein the at least one aperture comprises a first aperture and a second aperture that are longitudinally offset from one another.
127. The apparatus of aspect 126, wherein the orienting device comprises a radiopaque marker disposed between the first aperture and the second aperture.
128. The apparatus of aspect 122, wherein the orienting device comprises an orienting element.
129. The apparatus of aspect 128, wherein:
   the orienting element comprises a first inflatable member and a second inflatable member;
   the first inflatable member extending from a shaft of the orienting device in a first direction;
   **the second** inflatable member extending from the shaft in a second direction; and
   wherein the second direction is substantially opposite the first direction.
130. The apparatus of aspect 129, wherein:
   the orienting device defines a first aperture extending in a third direction through a wall of the orienting device; and
   the orienting device defines a second aperture extending in a fourth direction through a wall of the orienting device.
131. The apparatus of aspect 130, wherein the fourth direction is generally opposite the third direction.
132. The apparatus of aspect 130, wherein the fourth direction is generally perpendicular to the first direction and the second direction.
133. The apparatus of aspect 130, wherein the fourth direction is generally perpendicular to the first direction and the second direction.

## Claims

1. An apparatus for facilitating treatment via a vascular wall (22) defining a vascular lumen (30) containing an occlusion (36) therein, the apparatus comprising:
an intravascular device (106) having a lumen (122) and a distal portion, the distal portion including a first aperture (126) and a second aperture (128) that are both in fluid communication with the lumen (122); and
a reentry device (100) having a body (138) and a penetrator (140) extending distally beyond a distal end (132) of the body (138), wherein the body (138) is biased to assume a bent shape including a bend (130) in a natural state, wherein the body (138) has a distal leg (144) disposed distally of the bend (130) and a proximal leg (146) disposed proximally of the bend (130);
wherein the penetrator (140) extends beyond the distal end (132) of the body (138) by a distance (L1), and the distal leg (144) has a length (L2), wherein the length (L2) is greater than the distance (L1).

2. The apparatus of claim 1, wherein the reentry device (100) is configured to be advanced into the lumen (122) of the intravascular device (106), wherein the intravascular device (106) is configured to hold the reentry device (100) in a compressed state wherein the distal leg (144) is substantially aligned with the proximal leg (146).

3. The apparatus of claim 2, wherein in the natural state, the distal leg (144) forms an angle (A) with respect to the proximal leg (146), whereby the distal leg (144) of the reentry device (100) is configured to be advanced through each of the first and second apertures (126, 128).

4. The apparatus of claim 3, wherein the angle (A) is between about 120 degrees and about 150 degrees.

5. The apparatus of any preceding claim, wherein a diameter (D2) of the body (138) is greater than a diameter (D1) of the penetrator (140).

6. The apparatus of claim 5, wherein the diameter (D1) of the penetrator (140) is between about 0.0508 mm (0.0020 inches) and about 0.1397 mm (0.0055 inches), and the diameter (D2) of the body (138) is between 0.2032 mm (0.008 inches) and 0.381 mm (0.015 inches).

7. The apparatus of any preceding claim, wherein the first aperture (126) extends away from the lumen (122) in a first direction and the second aperture (128) extends away from the lumen (122) in a second direction, wherein the first direction is about 180 degrees from the second direction.

8. The apparatus of any one of claims 1-6, wherein the intravascular device (106) comprises an elongate shaft (120), a first balloon (152), and a second balloon (154).

9. The apparatus of claim 8, wherein the first balloon (152) extends from the elongate shaft (120) in a first direction and the second balloon (154) extends away from the elongate shaft (120) in a second direction, wherein the second direction is substantially opposite the first direction.

10. The apparatus of claim 9, wherein the first aperture (126) extends in a third direction and the second aperture (128) extends in a fourth direction, wherein the fourth direction is generally opposite the third direction.

11. The apparatus of claim 10, wherein the fourth direction is generally perpendicular to the first direction and the second direction.

12. The apparatus of any one of claims 8-11, wherein when the first balloon (152) and the second balloon (154) are inflated between an adventitia (24) of the vascular wall (22) and an intima (26) of the vascular wall (22), the intravascular device (106) will orient itself so that either the first aperture (126) or the second aperture (128) opens toward the vascular lumen (30).

13. The apparatus of any preceding claim, wherein the first aperture (126) and the second aperture (128) are longitudinally separated from one another.

14. The apparatus of any preceding claim, wherein the intravascular device (106) includes a first radiopaque marker (130A) located between the first aperture (126) and the second aperture (128).

15. The apparatus of claim 14, wherein a second radiopaque marker (130B) located distally of the second aperture (128).
